# EUROPÄISCHE PATENTANMELDUNG

(11) **EP 1 059 087 A1**
(43) Veröffentlichungstag der Anmeldung: **13.12.2000**
(21) Anmeldenummer: 99110895.2
(22) Anmeldetag: 08.06.1999
(51) Int. Cl.: A61K 38/17, C07K 14/47, C07K 16/18, G01N 33/564

(54) **Antigene von rheumatischen Autoimmunerkrankungen**

(71) Anmelder: Prehm, Peter, Prof. Dr., 48301 Nottuln (DE)
(72) Erfinder: Prehm, Peter, Prof. Dr., 48301 Nottuln (DE)
(74) Vertreter: Meyers, Hans-Wilhelm, Dr.Dipl.-Chem.

(57) **Zusammenfassung**

Verwendung von Reaktionsprodukten erhältlich durch Umsetzung von
- Proteinen und/oder Peptiden mit
- reaktiven Substanzen, die unter physiologischen Bedingungen unter Ausbildung kovalenter Bindungen mit funktionellen Gruppen der Proteine und/oder Peptide reagieren,
wobei die Reaktionsprodukte von Antikörpern von Patienten mit rheumatischen Autoimmunerkrankungen erkannt werden
als Antigene zur Diagnose und Therapie von rheumatischen Autoimmunerkrankungen, insbesondere rheumatischer Arthritis.

## Beschreibung

Die Erfindung betrifft Antigene von rheumatischen Autoimmunerkrankungen.

Über die Struktur und die Entstehung von Antigenen von chronisch entzündlichen Autoimmunkrankheiten, insbesondere rheumatischen Autoimmunerkrankungen, ist bisher wenig bekannt. Zu ihnen zählen die chronische rheumatische Arthritis und die juvenile chronische Arthritis, verschiedene Vasculitiden, rheumatisches Fieber, das Felty Syndrom, Morbus Bechterew, Lupus Erythematodes, Sklerodermie, Polymyalgia rheumatica, Psoriasis Arthritis und das Sjörgen Syndrom. Die meiste Information ist über die Entstehung von rheumatischer Arthritis verfügbar. Deswegen wird die Pathogenese dieser Autoimmunerkrankung stellvertretend für die anderen entzündlichen rheumatischen Autoimmunerkrankungen eingehender beschrieben.

Rheumatische Arthritis ist eine chronische Autoimmunerkrankung, die durch unbekannte Antigene verursacht und aufrecht erhalten wird (1,2). Die Entzündung beginnt in Gelenken und kann sich auf das Herz, die Lunge, die Augen, die Haut und die Blutgefäße ausbreiten. Die Pathogenese wird am besten durch "Hit and Run"-Mechanismus erklärt: Eine vorübergehende Infektion löst die chronische Entzündung aus (3). Es ist bekannt, daß eine Reaktive Arthritis, die durch Mikroorganismen ausgelöst wird, in etwa 30% zu einer chronischen rheumatischen Arthritis wird.

Für die chronische Entzündung sind Immunkomplexe nötig, die die Komplementkaskade, T-Zellen und Granulozyten aktivieren (4-12). Es gibt mehrere Methoden, diese Immunkomplexe aus Seren oder Synovialflüssigkeiten zu isolieren (13-15). Am besten hat sich die Fällung mit Polyethylenglykol-6000 bewährt. Die Analyse dieser Immunkomplexe hat gezeigt, daß zwei ähnliche Immunkomplexe in Patienten mit rheumatischer Arthritis vorkommen:

### 1. Rheumafaktoren:

Das sind Antikörper vom Typ IgG oder IgM, die an die schwere Kette von IgG binden und deshalb selbst aggregieren können. Die Aggregate aus IgG enthalten nur Rheumafaktoren und keine Antikörper mit anderen Spezifitäten. Deshalb aggregieren sie in konzentrierten Lösungen zu Dimeren, Tetrameren und Hexameren (16,17). In verdünnten Lösungen mit Dimeren binden sie kein C1q und aktivieren auch nicht das Komplement, weil dafür die Bindung eines Immunkomplexes von mindestens drei IgG-Molekülen erforderlich ist. Es hat sich herausgestellt, daß in 20% der Patienten mit rheumatischer Arthritis keine Rheumafaktoren vorhanden sind, und daß die Rheumafaktoren das Komplementsystem nicht aktivieren können. Deswegen können sie nicht für die Entstehung der chronischen Entzündung verantwortlich sein.

### 2. Andere Immunkomplexe:

Sie enthalten und aktivieren Komplement (10,18-21); sie haben Sedimentationskonstanten über 12S, sind reversibel durch Säure in monomere IgG dissoziierbar und besitzen eine andere Spezifität als Rheumafaktoren (22-24). Weil Antikörper aus diesen Immunkomplexen aber auch an IgG binden, muß eine Strukturveränderung des IgG für die Bindung verantwortlich sein (25). Die veränderte Struktur könnte durch freie Sauerstoffradikale erzeugt worden sein (26,27). Da Sauerstoffradikale bei der Entzündung durch aktivierte Granulozyten gebildet werden, wurde vermutet, daß sich diese Reaktion in der rheumatoiden Synovia selbst unterhält (28). Es ist bisher jedoch nicht gezeigt worden, daß sauerstoffradikal-verändertes IgG wirklich in den Immunkomplexen der rheumatoiden Synovialflüssigkeit vorkommt. Diese Hypothese erklärt auch nicht die Gewebsspezifität der Entzündung, die sich auf Hyaluronan-produzierendes Bindegewebe beschränkt.

Es fehlt nicht an Versuchen, Antigene bei Patienten mit rheumatischer Arthritis zu identifizieren und sie für die Diagnose und Therapie zu nutzen (29-55). Aber alle bisher gefundenen Antigene sind Epitope von nicht-modifizierten Proteinen. Im Verlauf der Entzündung kann es natürlich auch zur Antikörperbildung gegen viele andere Proteine kommen, die die Entzündung selbst nicht ausgelöst haben. Solche Proteine findet man aber nicht in den komplement-aktivierenden Immunkomplexen (18-21), und deswegen-können auch sie nicht an der Entstehung der chronischentzündlichen Autoimmunkrankheiten beteiligt sein.

Viele chronisch-rheumatische Autoimmunkrankheiten treten in Bindegeweben auf, die Hyaluronan produzieren. Hyaluronan ist ein natürlich vorkommendes Glucosaminoglycan mit alternierenden β1-3 glucuronischen und β1-4 glucosaminischen Bindungen. Das Molekulargewicht liegt normalerweise im Bereich von 50.000 bis 8.000.000 Da. Es wird von vielen Geweben gebildet und kommt in besonders hohen Konzentrationen in der Synovialflüssigkeit, in der Nabelschnur, im Glaskörper des Auges, in der Lunge, im Herzmuskel und in der Haut vor. Auch pathogene Bakterien wie Streptococcen der Gruppen A und C und Pasteurella produzieren eine Kapsel von Hyaluronan. Hyaluronan ist außerordentlich empfindlich gegenüber der Degradierung mit Sauerstoffradikalen. Die Sauerstoffradikale werden von aktivierten Granulozyten bei Entzündungsreaktionen gebildet, um Infektionen zu bekämpfen. Dabei können sie aber auch das eigene Gewebe schädigen. In Geweben mit hohem Hyaluronangehalt entstehen dabei radikalische Degradierungsprodukte aus Aldehyden, die ihrerseits wieder mit Aminogruppen von Proteinen und Peptiden reagieren können.

Der Synovialflüssigkeit verleiht Hyaluronan ihre außergewöhnlichen viskoelastischen Eigenschaften als Gleitmittel. Bei der Synovitis wird das mittlere Molekulargewicht von ca. 7x10⁶ auf die Hälfte reduziert (56). Die Degradierung erfolgt durch Sauerstoffradikale, die von infiltrierten Lymphozyten erzeugt werden und - kann durch Hemmstoffe der Radikalbildung verhindert werden. Besonders wirksam war DETAPAC, ein Eisenchelator, der die Haber-Weiss-Reaktion zur Bildung von Hydroxylradikalen hemmt (57). Außerdem besitzt Hyaluronan aus der Synovialflüssigkeit von Patienten mit rheumatischer Arthritis veränderte Eigenschaften. Es hat eine höhere Sedimentationsrate (58) und ist sehr fest an Protein gebunden (59-65). In der Synovialflüssigkeit kann Hyaluronan kovalent an IgG gebunden werden. Dadurch entstehen Immunkomplexe, die eine C1q-Bindungsaktivität besitzen (66). Diese IgG-Hyaluronan-Verbindungen machen nicht den Hauptanteil der Immunkomplexe aus.

Es ist bekannt, daß in der rheumatoiden Synovialflüssigkeit durch radikalische Degradierung aus Hyaluronan Carbonylgruppen entstehen (67,68). Ein Abbauprodukt des Hyaluronans ist L-threo-Tetradialdose (Synonym zu 2,3-Dihydroxy-1,4-butandial und Tartardialdehyd) (69). Dieses Produkt entsteht auch beim radikalischen Abbau von Ribose (70) oder Glucose (71).

Aufgabe der vorliegenden Erfindung ist die Bereitstellung neuer Arznei- und Diagnostikmittel.

Gelöst wird die Aufgabe durch die Verwendung von Reaktionsprodukten erhältlich durch Umsetzung von
- Proteinen und/oder Peptiden mit
- reaktiven Substanzen, die unter physiologischen Bedingungen unter Ausbildung kovalenter Bindungen mit funktionellen Gruppen der Proteine und/oder Peptide reagieren,
wobei die Reaktionsprodukte von Antikörpern von Patienten mit rheumatischen Autoimmunerkrankungen erkannt werden,
als Antigene zur Diagnose und Therapie von rheumatischen Autoimmunerkrankungen, insbesondere rheumatischer Arthritis.

Es wurde überraschenderweise gefunden, daß solche Reaktionsprodukte in den Immunkomplexen von Patienten mit rheumatischen Autoimmunerkrankungen vorkommen. Bevorzugte reaktive Substanzen sind solche die ein oxidatives Abbauprodukt eines Kohlenhydrates, bevorzugt von Hyaluronan sind. Entsprechende reaktive Substanzen können unterschiedlichste Molekulargewichte aufweisen, es ist jedoch bevorzugt, daß das Molekulargewicht kleiner 10.000 Da, noch mehr bevorzugt kleiner 1.000 Da ist. Auf der anderen Seite sind die Reaktionsprodukte bessere Antigene, wenn die reaktiven Substanzen eine gewisse Mindestgröße aufweisen. Es wird daher bevorzugt, daß diese Verbindungen mindestens zwei, bevorzugt mindestens drei, und noch mehr bevorzugt mindestens vier C-Atome aufweisen.

Besonders geeignete reaktive Substanzen weisen mindestens eine Carbonylfunktion, bevorzugt eine Aldehyd- oder Ketogruppe auf. Es ist dabei bevorzugt, daß die reaktive Substanz zwei oder mehr Carbonylfunktionen aufweist, da dies möglicherweise die Stabilität der Bindung der reaktiven Substanzen an die Peptide oder Proteine erhöht.

Bei dieser Reaktion entstehen zunächst Schiffsche Basen, die zu weiteren Verbindungen reagieren können. Mit L-threo-Tetradialdose entstehen zunächst Pyrrol-Derivate, die in Tautomerie mit Diketopyrrolidinen stehen und ebenfalls zu weiteren Produkten reagieren.

In einer bevorzugten Ausführungsform handelt es sich bei den reaktiven Substanzen um niedermolekulare, oxidative Abbauprodukte von Kohlenhydraten und zu diesen Abbauprodukten homologe Verbindungen. "Homologe Verbindungen" bezeichnen solche Verbindungen, bei denen funktionelle Gruppen entfernt oder hinzugefügt werden, ohne die Reaktionsmöglichkeiten mit den Peptiden und Proteinen auszuschließen. Handelt es sich bei dem oxidativen Abbauprodukt des Kohlenhydrates beispielsweise um eine Verbindung, die eine oder mehrere Hydroxygruppen aufweist, so ist ein Homolog eine Verbindung, die weniger oder keine Hydroxygruppen aufführt, beispielsweise 2,3-Dihydroxy-1,4-butandial (Tetradialdose) mit den homologen Verbindungen 2-Hydroxy-1,4-butandial und 1,4-Butandial.

Bevorzugte reaktive Substanzen genügen der allgemeinen Formel

R_{A}(Rₓ)ₓR_{B}

wobei
R_{A} und R_{B} unabhängig voneinander ausgewählt sind aus CH₃, CH=O, CH-OH, COOH, H und
jedes Rₓ unabhängig voneinander ausgewählt wird aus CH₂, CH-OH,
C=O oder einem Monosaccharid oder Oligosaccharid
x ausgewählt wird aus 0 bis 30, bevorzugt 0 bis 10 und mindestens eine der R_{A}, Rₓ und R_{B} eine Carbonylfunktion umfaßt.

Besonders bevorzugte reaktive Substanzen sind ausgewählt aus Tetradialdose, Glyoxal, Glyoxylat, Formaldehyd, Glutardialdehyd und Hydroxymalondialdehyd.

Bevorzugte Peptide oder Proteine sind Antikörper oder Fragmente von Antikörpern.

Auch die Reaktionsprodukte sind Bestandteil der Erfindung. Die erfindungsgemäßen Reaktionsprodukte sind dadurch erhältlich, daß man die reaktiven Substanzen, die unter physiologischen Bedingungen unter Ausbildung kovalenter Bindungen mit funktionellen Gruppen der Proteine und/oder Peptide reagieren, mit Proteinen und/oder Peptiden bei einem pH-Wert von 2 bis 10 bei einer Temperatur von 0 bis 50°C für 5 min bis 24 Stunden umsetzt.

Auch Antikörper, die die erfindungsgemäß verwendeten Reaktionsprodukte spezifisch erkennen, d.h. ein durch Umsetzung des entsprechenden Proteins oder Peptides mit den reaktiven Substanzen erhältliches Reaktionsprodukt mit einer Affinität binden, die mindestens zwei Größenordnungen größer ist als die Affinität zum nichtumgesetzten Protein oder Peptid, sind Bestandteil der Erfindung.

Sie sind erhältlich beispielsweise durch Immunisierung von Versuchstieren oder chromatographische Aufreinigung aus Körperflüssigkeiten.

### Rheumatische Arthritis

Die Erfindung wird unter Bezugnahme auf rheumatische Arthritis näher erläutert.

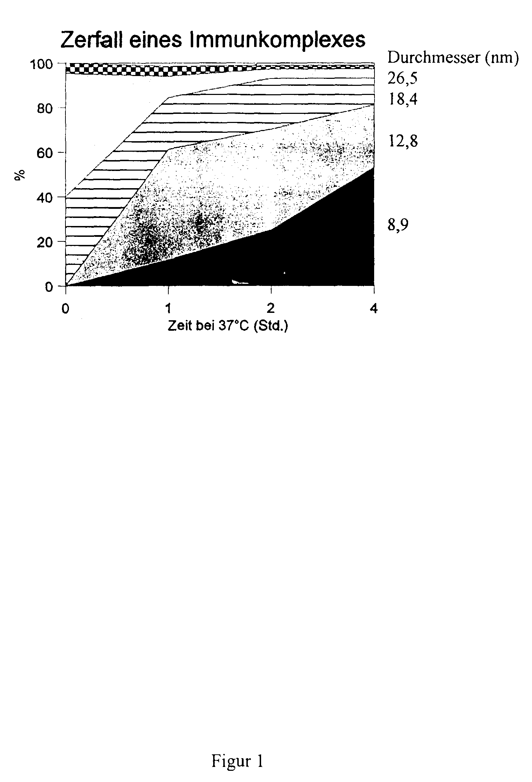

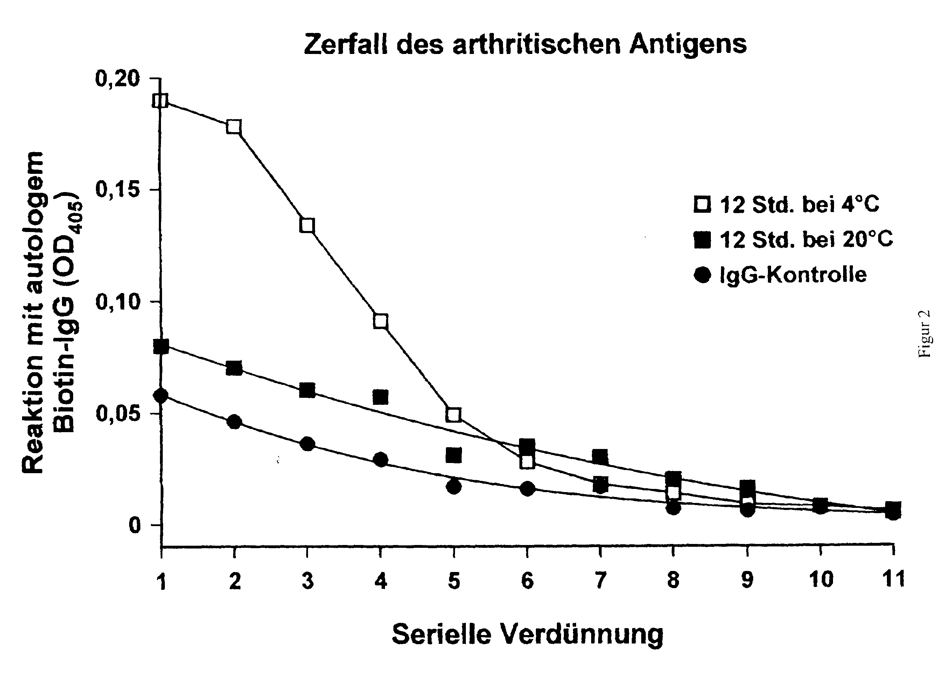

Immunkomplexe wurden aus Synovialflüssigkeiten von Patienten mit rheumatischer Arthritis durch Fällung mit Polyethylenglycol-6000 und anschließender Reinigung über Protein-A-Sepharose gewonnen. Sie enthalten IgG in elektrophoretisch homogener Form und können bis zu 20% des IgG in der Synovialflüssigkeit ausmachen. Sie unterscheiden sich aber von normalem IgG dadurch, daß sie unter physiologischen Bedingungen (pH 7.4, 0.15 M NaCl) Aggregate von 10 bis 50 nm Durchmesser bilden. Die Aggregation ist abhängig von der Konzentration des IgG und von der Durchführung der Präparation. Wenn die Präparation nicht bei 0°C innerhalb von 90 min erfolgt, wird die Neigung des IgG zur Selbstaggregation geringer. Dann bleibt eine geringere Bindungsaffinität, die den Rheumafaktoren ähnlich ist. Eine frisch isolierte Präparation bindet die Komplementkomponente C1q. Es hat sich weiter herausgestellt, daß diese Aggregate labil sind und mit einer Halbwertszeit von ca. 30 min bei Raumtemperatur zerfallen. Der Zerfall läßt sich im Nephelometer verfolgen (Figur 1). Somit ist auch nur eine Bindung von biotinyliertem IgG-Antikörper an autologes IgG-Antigen in einem ELISA-Test zu messen, wenn das IgG-Antigen frisch ist und bei 0°C gehalten wurde (Figur 2).

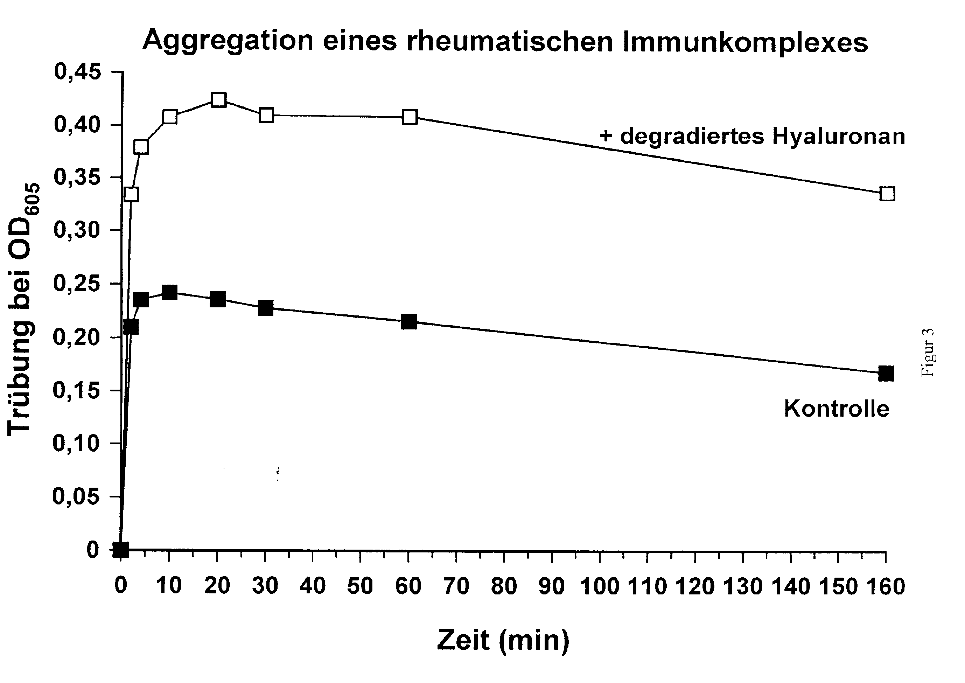

Die Bildung und der Zerfall von Aggregaten läßt sich durch Turbimetrie verfolgen, wenn ein IgG-Eluat aus der Protein-A-Sepharose Säule von pH 2.5 auf pH 7.0 neutralisiert wird. Dabei ist die Zunahme der Trübung wesentlich größer, wenn dem IgG vor der Aggregation eine Lösung von sauerstoffradikal-degradiertem Hyaluronan zugesetzt wurde (Figur 3). Das sauerstoffradikal-degradierte Hyaluronan erzeugt also weitere Antigene in der IgG-Präparation.

Eine sehr reaktive Komponente in sauerstoffradikal-degradiertem Hyaluronan ist L-threo-Tetradialdose. Diese Verbindung läßt sich auch aus Mannitol synthetisieren (72,73). Die Oxidation von 3,4-Monoaceton-mannitol durch Bleitetraacetat kann durch Natriumperjodat ersetzt werden. In Modellreaktionen setzt sich L-threo-Tetradialdose mit a-Acetyl-Lysin bei 37°C zu einem Reaktionsgemisch um, das ein Maximum der Ultraviolettabsorption bei 320 nm zeigt. Wenn das Verhältnis von a-Acetyl-Lysin zu L-threo-Tetradialdose über 1:1 erhöht wird, nimmt die Absorption bei 320 nm nicht weiter zu. Daraus kann man schließen, daß die Reaktionspartner in äquimolarem Verhältnis miteinander reagieren. Aus den Daten wurde eine molare Absorption von e = 281 berechnet. Ein ähnliches Absorptionsspektrum wurde von Dihydroxy-Pyrrolen beschrieben (74). Diese Verbindungen besitzen aber eine molare Absorption von e = 13.400. Daraus läßt sich folgern, daß bei der Umsetzung von L-threo-Tetradialdose mit a-Acetyl-Lysin nur zu einem Teil Dihydroxy-Pyrrole entstehen.

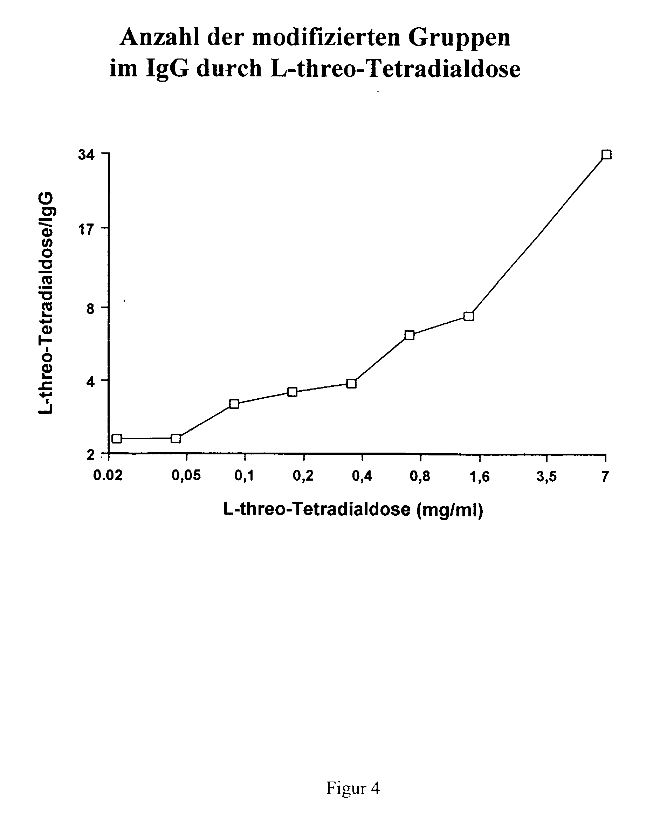

Die Umsetzung von normalem humanem IgG mit L-threo-Tetradialdose führt ebenfalls zu einer Absorption bei 320 nm. Das Ausmaß der IgG-Modifikation ist abhängig von den Konzentrationen. Eine IgG-Lösung mit 1 mg/ml bei pH 7.2 wird bei 37°C nach 16 Stunden mit steigenden Konzentrationen von L-threo-Tetradialdose zunehmend modifiziert (Figur 4). Durch Protease-Abbau des modifizierten IgG mit Pepsin und anschließender Trennung des Gemisches auf einer Reverse Phase-HPLC Säule lassen sich einzelne Peptide isolieren, die ebenfalls bei 320 nm absorbieren. Ähnliche Peptide mit einer Absorption bei 320 nm entstehen auch aus IgG, das aus Immunkomplexen der Synovialflüssigkeit von Patienten mit rheumatischer Arthritis isoliert wurden. Eine Aminosäuresequenzierung dieser Peptide zeigt folgende Sequenzen: AEV... und LFP.... Diese Sequenzen liegen in der schweren Kette des IgG AEVKKP... und LFPKPK..., unmittelbar vor den basischen Lysin-reichen Sequenzen. Die Lysine sind wahrscheinlich durch L-threo-Tetradialdose am Lysin modifiziert, was zu einem Abbruch der Sequenzierung führt.

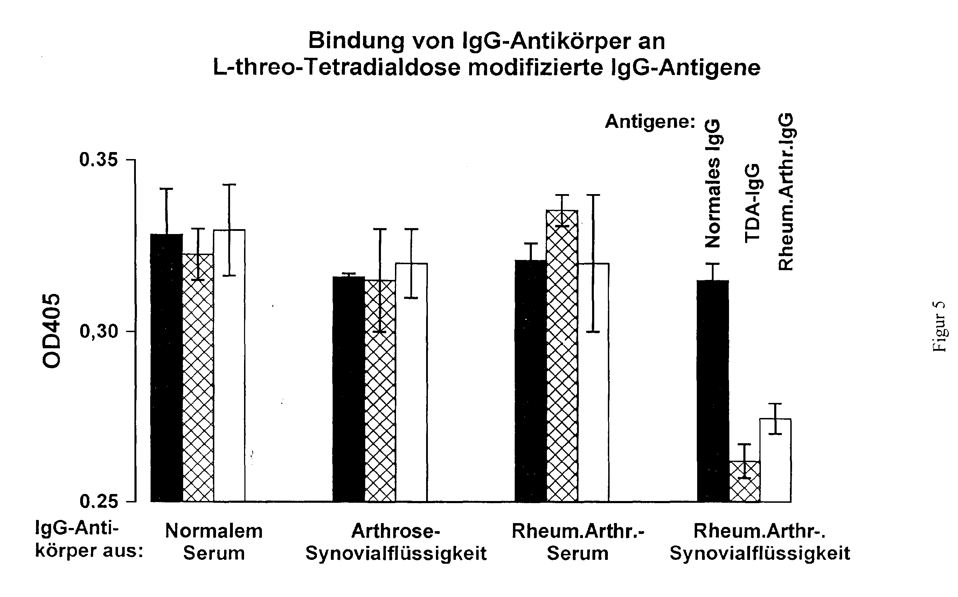

Das Reaktionsprodukt aus L-threo-Tetradialdose und IgG wird als ein Antigen von IgG-Antikörpern aus Synovialflüssigkeiten von Patienten mit rheumatischer Arthritis erkannt. Normales Humanserum oder Synovialflüssigkeiten von Patienten mit nicht-entzündlicher Arthrose reagieren dagegen nicht. Der Nachweis gelingt, wenn der Test schnell durchgeführt wird, um die Zersetzung des Antigens zu vermeiden. Dazu wird normales IgG, L-threo-Tetradialdose modifiziertes IgG oder IgG aus Immunkomplexen von rheumatischer Synovialflüssigkeit an Nitrocellulose in einem ELIFA-Gerät (Enzyme Linked Immno Flow Assay) adsorbiert und eine geeignete Verdünnung von Serum- oder Synovialflüssigkeit durch den Filter gesaugt. Nach dem Auswaschen von nicht-adsorbierten Komponenten wird der Filter mit einer Pufferlösung bei pH 2.5 in eine Mikrotiterplatte eluiert. Diese Mikrotiterplatte war vorher mit normalem humanem IgG beschichtet worden. Nach der Neutralisierung des Eluats in den Vertiefungen der Mikrotiterplatte auf pH 7.4 wird eine Lösung von Anti-human-IgG gekuppelter Peroxidase hinzugefügt. Das eluierte IgG konkurriert nun mit dem voradsorbierten IgG um die Anti-human-IgG gekuppelte Peroxidase und läßt sich in einer anschließenden Farbreaktion bestimmen. Das Resultat zeigt, daß Antikörper nur in rheumatischer Synovialflüssigkeit vorhanden sind. Sie binden genau so gut an L-threo-Tetradialdose modifiziertes IgG, wie an IgG aus rheumatischen Immunkomplexen, aber nicht an normales IgG (Figur 5). Damit ist dieser Test spezifisch für Patienten mit rheumatischer Arthritis, und er beweist, daß sowohl die Antikörper als auch die Antigene in der Synovia von Patienten mit rheumatischer Arthritis gebildet werden.

Dieses Testprinzip läßt sich auch auf ein einfaches immunochromatographisches oder "Dipstick-Verfahren" übertragen, wobei die Empfindlichkeit soweit erhöht wird, daß die Antikörper auch in Seren von Patienten mit rheumatischer Arthritis nachgewiesen werden können.

### Beispiele

### 1. Erzeugung radikalischer Degradierungsprodukte von Kohlenhydraten

Es gibt viele Reagenzien, die Hydroxylradikale erzeugen können (75). Vorteilhaft ist die Kupfersulfat-katalysierte Reaktion aus Wasserstoffperoxid. Als Substrate eignen sich Monosaccharide wie Glucose, Oligosaccharide Polysaccharide, insbesonders Hyaluronan. Hier wird beispielhaft die Degradierung von Hyaluronan beschrieben. Eine Lösung von 20 mg von Hyaluronan (z.B. Healon® von Pharmacia) in 2 ml phosphatgepufferte NaCl-Lösung wird mit 0.2 ml einer 55 mM Kupfersulfat-Lösung und 0.2 ml einer 10 mM Wasserstoffperoxid-Lösung versetzt und im Glashomogenisator gemischt. Nach einer Inkubation bei 37°C für 16 Stunden kann die Lösung zur Modifikation von Proteinen oder Peptiden eingesetzt werden.

### 2. Herstellung von Antigenen entzündlich-rheumatischer Autoimmunerkrankungen

Degradiertes Hyaluronan oder L-threo-Tetradialdose wird nun verwendet, um Antigene zu erzeugen. Dabei können sowohl Peptide als auch Proteine, insbesondere IgG eingesetzt werden. Hier wird beispielhaft die Modifikation von IgG beschrieben.

Es wird eine Lösung von Proteinen oder Peptiden hergestellt und mit einer Lösung von degradiertem Hyaluronan oder L-threo-Tetradialdose zu einer Endkonzentration von 1 mg/ml bis 20 mg/ml gemischt und für 5 min bis 24 Stunden bei einem pH-Wert zwischen 2 und 10 inkubiert. Die Temperatur kann zwischen 0°C und 50°C liegen. Vorteilhaft ist eine Temperatur von 37°C, eine Inkubationszeit von 1 Stunde bei einem pH-Wert von 7.4 und eine Protein- oder Peptid-Konzentration von 1 mg/ml. Danach wird die Lösung eingefroren und bei -70°C gelagert.

### Diagnostische und therapeutische Verwendung der modifizierten Peptide und Proteine

### (1) Diagnose entzündlich-rheumatischer Autoimmunerkrankungen

Es gibt bisher keinen zuverlässigen biochemischen oder immunologischen Nachweis für rheumatische Arthritis oder andere rheumatische Autoimmunerkrankungen, weil sowohl die Pathogenese als auch die treibenden Antigene der chronischen Entzündung unbekannt sind. Die durch die reaktiven Substanzen modifizierten Proteine und Peptide lassen sich in bekannten immunologischen Testmethoden verwenden, um in Seren oder Synovialflüssigkeiten die Titer von Autoantikörpern gegen diese Proteine zu bestimmen. Um Antikörper gegen labile Antigene zu erfassen eignen sich besonders schnelle Testmethoden, wie ELIFA, Dipstick, Plasmon Resonanz, immunochromatographische und nephelometrische Methoden.

### (2) Spezifität der Antikörper

Die reaktiven Substanzen können mit einer Vielzahl verschiedener Proteine und Peptide reagieren, vornehmlich mit solchen, die wie das IgG basischer sind als andere Serumproteine oder mit Proteinen, die sich in räumlicher Nachbarschaft zum degradierten IgG befinden, wie Hyaluronan-bindende Proteine. Darüber hinaus können verschiedene Gruppen, vornehmlich Lysine, reagieren. Dadurch werden unterschiedliche Autoantikörper gebildet, die zu verschiedenen Krankheitssymptomen führen. Durch die Auswahl der zu modifizierenden Proteine oder Peptide als Antigen in den immunologischen Testmethoden kann die Spezifität der Autoantikörper untersucht werden.

### (3) Therapie entzündlich-rheumatischer Autoimmunerkrankungen

Das Ziel der Therapie ist die Adsorption der Autoantikörper aus der Zirkulation oder die Erzeugung von Toleranz. Die Adsorption der Autoantikörper läßt sich durch Plasmapherese an matrixgebundenen Proteinen erreichen, die durch reaktive Substanzen modifiziert worden sind. Eine Toleranz könnte durch orale Verabreichung von antigenen Proteinen oder durch Behandlung mit antigenen Peptidfragmenten erzeugt werden.

### (4) Herstellung monoklonaler Antikörper

Monoklonale Antikörper können bei der Untersuchung der Antigenstrukturen wichtige Hilfsmittel darstellen. Zur Immunisierung, Isolierung und Charakterisierung spezifischer Klone können Proteine oder Peptide eingesetzt werden, die durch reaktive Substanzen modifiziert worden sind.

### (5) Isolierung spezifischer Antikörper aus der Blutzirkulation oder der Synovialflüssigkeit

Patienten mit entzündlich-rheumatischen Autoimmunerkrankungen unterscheiden sich in der Spezifität der Autoantikörper. Spezifische Autoantikörper können isoliert werden durch Adsorption an Matrix-gebundene Proteine, die durch reaktive Substanze modifiziert worden sind.

### Literatur

1. Harris, E. D. Pathogenesis of Rheumatoid Arthritis. Kelley, W. N., Harris, E. D., Ruddy, E. D., and Sledge, C. B. Textbook of Rheumatology. [54]. 1989. Philadelphia, W.B. Saunders.
2. Deicher, H. Pathomechanismen entzündlicher rheumatischer Erkrankungen bei Mensch und Tier. 1989. Weinheim, VCH. Sonderforschungsbereiche der DFG.
3. Burmester, G. R. Hit and run or permanent hit? Is there evidence for a microbiological cause of rheumatoid arthritis? Journal of Rheumatology 18, 1443-1447. 1991.
4. Winchester, R. J., Agnello, and V Kunkel, H. G. Gamma globin complexes in synovial fluid of patients with rheumatiod arthritis. Partial characterization and relationship to lowered complement levels. Clin.Exp.Immunol. 6, 689-706. 1970.
5. Hedburg, H. Studies on the depressed complement activity in synovial fluid in adult rheumatoid arthritis. Acta Rheum.Scand. 9, 165-165. 1963.
6. Pekin, T. J. and Zvaifler, N. J. Haemolytic complement in synovial fluid. Journal of Clinical Investigation 43, 1371-1371. 1964.
7. Morgan, P., Daniels, R. H., and Williams BD. Measurement of terminal complement complexes in rheumatoid arthritis. Journal of Experimental Immunology 73, 473-478. 1988.
8. Magged, R. A., Kirwan, J. R., Thompson, P. W., McCarty, D. A., and Holborow, E. J. Characterization of the size and composition of circulating immune complexes in patients with rheumatoid arthrits. Ann.Rheum.Dis. 50, 231-236. 1991.
9. Hain, N., Alsalameh, S., Bertling, W. M., Kalden, J. R., and Burmester, G. R. Stimulation of rheumatoid synovial and blood T cells andlines by synovial fluid and interleukin-2: characterization of clones and recognition of a co-stimulatory effect. Rheumatol.Int. 10, 203-210. 1990.
10. Bedwell, A. E., Elson, C. J., Carter, S. D., Dieppe, P. A., and Hutton, C. W. Czudek-R. Isolation and analysis of complement activating aggregates from synovial fluid of patients with rheumatoid arthritis using monoclonal anti-C3d antibodies. Ann.Rheum.Dis. 46, 55-64. 1987.
11. Gale, R., Bertouch, J. V., Bradley, J., and Roberts Thomson, P. J. Direct activation of neutrophil chemiluminescence by rheumatoid sera and synovial fluid. Ann.Rheum.Dis. 42, 158-162. 1983.
12. Robinson, J. J., Watson, F., Phelan, M., Bucknall, R. C., and Edwards, S. W. Activation of neutrophils by soluble and insoluble immunoglobulin aggregates from synovial fluid of patients with rheumatoid arthritis. Ann.Rheum.Dis. 52, 347-353. 1993.
13. Jones, V. E. and Orlans, E. Isolation of immune complexes and characterization of their constituent antigens and antibodies in some human diseases: a review. Journal of Immunological Methods 44, 249-270. 1981.
14. Virella, G., Kilpatrick, J. M., Chenais, F., and Fudenberg, H. H. Isolation of soluble immune complexes from human serum: combined use of polyethylene glycol precipitation, gel filtration, and affinity chromatography on Protein A-Sepharose. Methods in Enzymology 74, 644-663. 1981.
15. Moore, T. L., Sheridan, P. W., Zuckner, J., and Dorner, R. W. Separation and characterization of immune complexes containing 19S IgM rheumatoid factor-IgG in juvenile arthritis. Arthritis and Rheumatism 26, 165-169. 1983.
16. Pope, R. M., Teller, D. C., and Mannik, M. The molecular basis of self-association of antibodies of IgG (Rheumatoid factors) in rheumatoid arthritis. Proc.Natl.Acad.Sci.USA 71, 517-521. 1974.
17. Pope, R. M., Teller, D. C., and Mannik, M. The molecular basis of self-association of IgG-Rheumatoid factors. Journal of Immunology 115, 365-373. 1975.
18. Male, D. and Roitt, I. M. Analysis of the components of immune complexes. Molecular.Immunology 16, 197-203. 1979.
19. Male, D., Roitt, I. M., and Hay, F. C. Analysis of immune complexes in synovial effusions of patients with rheumatoid arthritis. Clin.Exp.Immunol. 39, 297-306. 1980.
20. Male, D. K. and Roitt, I. M. Molecular analysis of complement-fixing rheumatoid synovial fluid immune complexes. Clin.Exp.Immunol. 46, 521-529. 1981.
21. Elson, C. J., Carter, S. D., Cottrell, B. J., Scott, D. G. I., and Bacon, P. A. Wallington-TB. Complement activating properties of complexes containing rheumatoid factor in synovial fluids and sera from patients with rheumatoid arthritis. Clin.Exp.Immunol. 59, 285-292. 1985.
22. Winchester, R. J. Charakterization of IgG complexes in patients with rheumatoid arthritis. Ann.N.Y.Acad.Sci. 256, 73-81. 1975.
23. Gustafson, S., Björkman, T., and Westlin, J.-E. Labelling of high molecular weight hyaluronan with ¹²⁵I-tyrosine: Studies *in vitro* and *in vivo* in the rat. Glycoconjugate J. 11, 608-613. 1994.
24. Robbins, D. L., Moore, T. L., Carson, D. A., and Vaughan, J. H. Relative reactivities of rheumatoid factors in serum and cells: evidence for a selective deficiency in serum rheumatoid factor. Arthritis and Rheumatism 21, 820-826. 1978.
25. Carter, S. D., Makh, S. R., Ponsford, F. M., and Elson, C. J. Rheumatoid factor has increased reactivity with IgG from synovial fluids of patients with rheumatoid arthritis and osteoarthritis. Br.J.Rheumatol. 28, 233-238. 1989.
26. Lunec, J., Griffiths, H. R., and Brailsford, S. Oxygen free radicals denature human IgG and increase its reactivity with rheumatoid factor antibody. Scand.J.Rheumatol.Suppl. 75, 140-147. 1988.
27. Griffiths, H. R. and Lunec, J. Effect of polymorph derived oxidants on IgG in relation to rheumatoid factor binding. Scand.J.Rheumatol.Suppl. 75, 148-156. 1988.
28. Lunec, J., Blake, D. R., and McCleary, S. J. Self-perpetuating mechanisms of immunoglobulin G aggregation in rheumatoid arthritis. Journal of Clinical Investigation 76, 2084-2090. 1985.
29. Serre, Guy, Girbal-Neuhauser, Elisabeth, Vincent, Christian, Simon, Michel, Sebbag, Mireille, Dalbon, Pascal, Jolivet-Reynaud, Colette, Arnaud, Michel, and Jolivet, Michel. Citrulline-containing antigens derived from filaggrin and their use for diagnosing rheumatoid polyarthritis. Biomerieux, Fr, Serre, Guy, Girbal-Neuhauser, Elisabeth, Vincent, Christian, Simon, Michel, Sebbag, Mireille, Dalbon, Pascal, Jolivet-Reynaud, Colette, Arnaud, Michel, and Jolivet, Michel. WO 97-FR1541[WO 9808946 A1]. 1998. PCT Int. Appl., 37 pp. CODEN: PIXXD2. 1-9-1997.
30. Carson, Dennis A. and Albani, Salvatore. Antigens for use in inducing immune tolerance to arthritogenic peptides and protection against rheumatoid arthritis. Regents of the University of California, USA. WO 97-US2957[WO 9734002 A1]. 1997. PCT Int. Appl., 44 pp. CODEN: PIXXD2. 20-2-1997.
31. Menard, Henri A. and Despres, Normand. Antigen-antibody system specific for rheumatoid arthritis: the sa system. Menard, Henri A. and Despres, Normand. CA 95-2161205[CA 2161205 AA]. 1997. Can. Pat. Appl., 47 pp. CODEN: CPXXEB. 23-10-1995.
32. Osaki, Shoichi, Tanaka, Masao, Kishimura, Masaaki, Nakao, Kazuwa, and Osakada, Fumio. Cloning of cDNA for autoantigens from patients with rheumatoid arthritis and use as diagnostic markers. Kaneka, Corporation, Osaki, Shoichi, Tanaka, Masao, Kishimura, Masaaki, Nakao, Kazuwa, and Osakada, Fumio. WO 96-JP3250[WO 9717441 A1]. 1997. PCT Int. Appl., 60 pp. CODEN: PIXXD2. 6-11-1996.
33. Nakagami, Tatsuyoshi Di, Matsumoto, Takashi, Taguchi, Yasuki, Fujita, Kotaro, Ametani, Akio, and Kaminogawa, Syuichi. Oral remedy for rheumatoid arthritis and functional food. Nippon Meat Packers, Inc. Japan, Nakagami, Masayo, Matsumoto, Takashi, Taguchi, Yasuki, Fujita, Kotaro, Ametani, Akio, and Kaminogawa, Syuichi. WO 96-JP1623[WO 9641644 A1]. 1996. PCT Int. Appl., 31 pp. CODEN: PIXXD2. 13-6-1996.
34. Prakash, Ramesh K. Recombinant antigen for diagnosing rheumatoid arthritis. Theratech, I. US 94-364081[US 5585464 A]. 1997. U.S., 9 pp. Cont.-in-part ofU.S. 5,395,753. CODEN: USXXAM. 27-12-1994.
35. Prakash, Ramesh K. Recombinant rheumatoid arthritis IgM-associated antigen (RAMA) for diagnosing rheumatoid arthritis. Theratech, I. WO 95-US16558[WO 9620213 A1]. 1996. PCT Int. Appl., 28 pp. CODEN: PIXXD2. 15-12-1995.
36. Rothbard, Jonathan, Fugger, Lars Henrik, and Sonderstrup-McDevitt, Grete. Peptides (collagen type II fragments), therapeutic compositions, and methods for administering compositions for treating rheumatoid arthritis. Immulogic, Pharmaceutical Corporation. WO 96-US206[WO 9620950 A2]. 1996. PCT Int. Appl., 45 pp. CODEN: PIXXD2. 4-1-1996.
37. Wagatsuma, Masako, Kimura, Michio, Watanabe, Hiroshi, and Takeuchi, Fujio. Method of detecting autoantibody present in the serum of rheumatic. Hoechst, Japan Limited. WO 95-JP1776[WO 9607914 A1]: 1996. PCT Int. Appl., 32 pp. CODEN: PIXXD2. 7-9-1995.
38. Uenokawa, Shuichi, Ametani, Akio, Yamura, Toshuki, Hisatsune, Tatsuhiro, and Matsumoto, Takayuki. Oral tolerogen compositions containing type II collagen-derived oligopeptide for the treatment of rheumatoid arthritis. Nippon, Hamu Kk and Sumitomo, Electric, I. JP 93-311231[JP 07138187 A2]. 1991. Jpn. Kokai Tokkyo Koho, 6 pp. CODEN: JKXXAF. 16-11-1993.
39. Praskash, Ramesh K. IgM-specific recombinant rheumatoid arthritis antigen for diagnosis. Theratech, I. WO 94-US689[WO 9419374 A1]. 1994. PCT Int. Appl., 37 pp. CODEN: PIXXD2. 18-1-1994.
40. Serre, Guy, Somme, Gerard, and Vincent, Christian. Antigens recognized by rheumatoid polyarthritis autoantibodies, their preparation and applications. Clonatec, S. A. EP 92-401185[EP 511116 A1]. 1992. Eur. Pat. Appl., 38 pp. CODEN: EPXXDW. 24-4-1992.
41. Okada, Yasunori, Shinmei, Masayoshi, Hayakawa, Taro, Iwata, Kazushi, Korin, Yumi, Kodama, Shuji, and Yoshida, Shinichi. Antihuman stromelysin monoclonal antibody and its use in diagnosis of rheumatoid arthritis by enzyme immunoassay. Fuji, Yakuhin Kogyo. WO 92-JP41[WO 9213096 A1]. 1992. PCT Int. Appl., 33 pp. CODEN: PIXXD2. 21-1-1992.
42. Stanworth, Denis Raymond, Lewin, Ian Victor, and Nayyar, Sarita. Immunodiagnostic assay for rheumatoid arthritis diagnosis. National Research Development Corp., UK. WO 91-GB821[WO 9119001 A1]. 1991. PCT Int. Appl., 42 pp. CODEN: PIXXD2. 24-5-1991.
43. Carson, Dennis A. and Roudier, Jean. Rheumatoid arthritis vaccine. Scripps Clinic and Research Foundation, USA. WO 90-US3038[WO 9014835 A1]. 1990. PCT Int. Appl., 30 pp. CODEN: PIXXD2. 31-5-1990.
44. Stetler, Dean Allen. Detection of RNA polymerase antigen and/or antibody in body fluids for diagnosing and monitoring rheumatic diseases. University, of Kansas. EP 90-312865[EP 430642 A1]. 1991. Eur. Pat. Appl., 16 pp. CODEN: EPXXDW. 27-11-1990.
45. Hayakawa, Taro, Kodama, Shuji, Iwata, Kazushi, Kishi, Junichi, Yamashita, Kyoko, and Iwata, Hisashi. Collagenase inhibitor determination in body fluids by EIA for diagnosing chronic articular rheumatism. Fuji Chemicals Industrial Co., Ltd. Japan. WO 88-JP846[WO 8902078 A1]. 1989. PCT Int. Appl., 45 pp. CODEN: PIXXD2. 25-8-1988.
46. Yamanaka, Naoki and Yoshida, Makoto. Substantially pure rheumatoid arthritis specific protein and antibody against it. Asahi Medical Co., Ltd. Japan and Medics, Co. EP 85-111630[EP 175310 A1]. 1986. Eur. Pat. Appl., 75 pp. CODEN: EPXXDW. 13-9-1985.
47. Jung, F., Neuer, G., and Bautz, F. A. Antibodies against a peptide sequence located in the linker region of the HMG-1/2 box domains in sera from patients with juvenile rheumatoid arthritis. Arthritis and Rheumatism 40, 1803-1809. 1997.
48. Nesher, G. Antiperinuclear factor - A specific marker for rheumatoid arthritis. Isr.J.Med.Sci. 33, 267-268. 1997.
49. Bonagura, V. R., Agostino, N., Borretzen, M., Thompson, K. M., Natvig, J. B., and Morrison, S. L. Mapping IgG epitopes bound by rheumatoid factors from immunized controls identifies disease-specific rheumatoid factors produced by patients with rheumatoid arthritis. Journal of Immunology 160, 2496-2505. 1998.
50. Schellekens, G. A., De Jong, B. A. W., Van den Hoogen, F. H. J., Van de Putte, L. B. A., and Van Venrooij, W. J. Citrulline is an essential constituent of antigenic determinants recognized by rheumatoid arthritis-specific autoantibodies. Journal of Clinical Investigation 101, 273-281. 1998.
51. Hain, N. A. K., Stuhlmüller, B., Hahn, G. R., Kalden, J. R., Deutzmann, R., and Burmester, G. R. Biochemical characterization and microsequencing of a 205- kDa synovial protein stimulatory for T cells and reactive with rheumatoid factor containing sera. Journal of Immunology 157, 1773-1780. 1996.
52. Dybwad, A., Forre, O., Natvig, J. B., and Sioud, M. Structural characterization of peptides that bind synovial fluid antibodies from RA patients: A novel strategy for identification of disease-related epitopes using a random peptide library. Clin.Immunol.Immunopathol. 75, 45-50. 1995.
53. Dybwad, A., Forre, O., and Sioud, M. Increased serum and synovial fluid antibodies to immunoselected peptides in patients with rheumatoid arthritis. Ann.Rheum.Dis. 55, 437-441. 1996.
54. Peterson, C., Malone, C. C., and Williams, R. C. Rheumatoid-factor-reactive sites on CH3 established by overlapping 7-mer peptide epitope analysis. Mol.Immunol. 32, 57-75. 1995.
55. Artandi, S. E., Calame, K. L., Morrison, S. L., and Bonagura, V. R. Monoclonal IgM rheumatoid factors biond IgG at a discontinous epitope comprised of amino acid loops from heavy-chain constant- region domains 2 and 3. Proceedings of the National Academy of Sciences USA [89], 94-98 X. 1992.
56. Dahl, L. B., Dahl, I. M., Engstrom Laurent, A., and Granath, K. Concentration and molecular weight of sodium hyaluronatein synovial fluid from patients with rheumatoid arthritis and other arthropathies. Ann.Rheum.Dis. 44, 817-822. 1985.
57. Schenck, P., Schneider, S., Miehlke, R., and Prehm, P. Synthesis and degradation of hyaluronate by synovia from patients with rheumatoid arthritis. Journal of Rheumatology 22, 400-405. 1995.
58. Johnston, J. P. A comparison of the properties of hyaluronic acid from normal and pathological human synovial fluids. Biochemical Journal 626-633, -633. 1955.
59. Scher, I. and Hamerman, D. Isolation of human synovial-fluid hyaluronate by density-gradient ultracentrifugation and evaluation of its protein content. Biochemical Journal 126, 1073-1080. 1972.
60. Hamerman, D. and Sandson, J. Unusual properties of hyaluronate protein isolated from pathological synovial fluids. Journal of Clinical Investigation 42, 1882-1889. 1963.
61. Sandson, J. and Hamerman, D. Binding of an alpha globin to hyaluronate protein in pathological synovial fluid. Science 146, 70-71. 1964.
62. Hutadilok, N., Ghosh, P., and Brooks, P. M. Binding of haptoglobin, inter-alpha-trypsin inhibitor, and alpha 1 proteinase inhibitor to synovial fluid hyaluronate and the influence of these proteins on its degradation by oxygen derived free radicals. Ann.Rheum.Dis. 47, 377-385. 1988.
63. Huang, L., Yoneda, M., and Kimata, K. A serum-derived hyaluronan-associated protein (SHAP) is the heavy chain of the inter alpha-trypsin inhibitor. Journal of Biological Chemistry 268, 26725-26730. 1993.
64. Wisniewski, H. G., Burgess, W. H., Oppenheim, J. D., and Vilcek, J. TSG-6, an arthritis-associated hyaluronan binding protein,forms a stable complex with the serum proteininter-alpha-inhibitor. Biochemistry 33, 7423-7429. 1994.
65. Jessen, T. E., Odum, L., and Johnsen, A. H. *In vivo* binding of human inter-a-trypsin inhibitor free heavy chains to hyaluronic acid. Biol.Chem.Hoppe Seyler 375, 521-526. 1994.
66. Prehm, P. Synovial hyaluronate in rheumatoid arthritis binds C1q and is covalently bound to antibodies: a model for chronicity. Ann.Rheum.Dis. 54, 408-412. 1995.
67. Grootveld, M., Henderson, E. B., Farrell, A., Blake, D. R., and Parkes, H. G. Haycock-P. Oxidative damage to hyaluronate and glucose in synovial fluid during exercise of the inflamed rheumatoid joint. Detection of abnormal low-molecularmass metabolites by proton-n.m.r. spectroscopy. Biochemical Journal 273, 459-467. 1991.
68. Chapman, M. L., Rubin, B. R., and Gracy, R. W. Increased carbonyl content of proteins in synovial fluidfrom patients with rheumatoid arthritis. Journal of Rheumatology 16, 15-18. 1989.
69. Uchiyama, H., Dobashi, Y., Ohkouchi, K., and Nagasawa, K. Chemical change involved in the oxidative reductive depolymerization of hyaluronic acid. Journal of Biological Chemistry 265, 7753-7759. 1990.
70. von Sonntag C and Dizdaroglu, M. The reactions of OH radicals with D-ribose in deoxygenated and oxygenated aqueous solution. Carbohydr.Res. 58, 21-30. 1977.
71. Schuchmann, M. N and von Sonntag C. Radiation chemistry of carbohydrates. Part 14. Hydroxyl radical induced oxidation of D-glucose in oxygenated aqueous solution. J.Chem.Soc.Perkin 2. 1958-1963. 1977.
72. Wiggins, L. F. The acetone derivatives of hexahydric alcohols. Part I. Tracetone mannitol and its conversion into d-arabinose. J.Chem.Soc. 13-15. 1946.
73. Stern, R. and Wassermann, H. H. Synthesis of (-)-6-exo,7-endo-Didhydroxy-3-tropanone, an optically active product from a Robinson-Mannich condensation. J.Org.Chem. 24, 1689-1691. 1959.
74. Vaughan, W. R. and Petersen, J. 2,3-Pyrrolidinediones. I Preparationd and structure. J.Org.Chem. 18, 382-392. 1953.
75. Wong, S. F., Halliwell, B., Richmond, R., and Skowroneck, W. R. The role of superoxide and hydroxyl radicals in the degradation of hyaluronic acid induced by metal ions and by ascorbic acid. J.Inorg.Biochem. 14, 127-134. 1981.

## Patentansprüche

1. Verwendung von Reaktionsprodukten erhältlich durch Umsetzung von
• Proteinen und/oder Peptiden mit
• reaktiven Substanzen, die unter physiologischen Bedingungen unter Ausbildung kovalenter Bindungen mit funktionellen Gruppen der Proteine und/oder Peptide reagieren,
wobei die Reaktionsprodukte von Antikörpern von Patienten mit rheumatischen Autoimmunerkrankungen erkannt werden
als Antigene zur Diagnose und Therapie von rheumatischen Autoimmunerkrankungen, insbesondere rheumatischer Arthritis.

2. Verwendung nach Anspruch 1, dadurch gekennzeichnet, daß die reaktive Substanz ein oxidatives Abbauprodukt eines Kohlenhydrates ist.

3. Verwendung nach einem der Ansprüche 1 oder 2, dadurch gekennzeichnet, daß die reaktive Substanz ein oxidatives Abbauprodukt von Hyaluronan ist.

4. Verwendung nach einem der Ansprüche 1 bis 3, dadurch gekennzeichnet, daß die reaktive Substanz mindestens zwei C-Atome und/oder ein Molekulargewicht kleiner 10.000 Da aufweist.

5. Verwendung nach einem der Ansprüche 1 bis 4, dadurch gekennzeichnet, daß die reaktive Substanz mindestens eine Carbonylfunktion, bevorzugt eine Aldehyd- oder Ketogruppe aufweist.

6. Verwendung nach einem der Ansprüche 1 bis 5, dadurch gekennzeichnet, daß die reaktive Substanz ausgewählt ist aus Tetradialdose, Glyoxal, Glyoxylat, Formaldehyd, Glutardialdehyd, Hydroxymalondiealdehyd.

7. Verwendung nach einem der Ansprüche 1 bis 6, dadurch gekennzeichnet, daß das Peptid oder Protein ein Antikörper oder ein Fragment eines Antikörpers ist.

8. Reaktionsprodukt erhältlich durch Umsetzung von Proteinen und/oder Peptiden mit reaktiven Substanzen, die oxidative Abbauprodukte des Hyaluronans sind, wobei diese mindestens zwei C-Atome und ein Molekulargewicht kleiner 10.000 Dalton aufweisen.

9. Reaktionsprodukt nach Anspruch 8, dadurch gekennzeichnet, daß die reaktive Substanz ausgewählt wird aus Tetradialdose, Glyoxal, Glyoxylat, Glutardialdehyd, Hydroxymalondialdehyd.

10. Verfahren zur Herstellung von Reaktionsprodukten nach einem der Ansprüche 8 oder 9, dadurch gekennzeichnet, daß man die reaktiven Substanzen, die unter physiologischen Bedingungen unter Ausbildung kovalenter Bindungen mit funktionellen Gruppen der Proteine und/oder Peptide reagieren, mit Proteinen und/oder Peptiden bei einem pH-Wert von 2 bis 10 bei einer Temperatur von 0 bis 50°C für 5 min bis 24 Stunden umsetzt.

11. Antikörper, dadurch gekennzeichnet, daß sie spezifisch Reaktionsprodukte nach einem der Ansprüche 8 oder 9 erkennen.
